(19)

Europäisches Patentamt
European Patent Office
Office européen des brevets

(11)  EP 2 165 647 A1

(12)  EUROPEAN PATENT APPLICATION

(43) Date of publication:
    24.03.2010  Bulletin 2010/12

(51) Int Cl.:
    *A61B 5/00* (2006.01)     *G01N 21/47* (2006.01)
    *G01N 21/49* (2006.01)

(21) Application number: 08019605.8

(22) Date of filing: 10.11.2008

(84) Designated Contracting States:
    AT BE BG CH CY CZ DE DK EE ES FI FR GB GR
    HR HU IE IS IT LI LT LU LV MC MT NL NO PL PT
    RO SE SI SK TR
    Designated Extension States:
    AL BA MK RS

(30) Priority:  13.08.2008  EP 08014407

(71) Applicant: Siemens Aktiengesellschaft
    80333 München (DE)

(72) Inventors:
    • Avramov, Latchezar
      1113 Sofia (BG)
    • Bliznakova, Irina
      1225 Sofia (BG)

    • Borisova, Ekaterina
      1387 Sofia (BG)
    • Dreischuh, Tanja
      1000 Sofia (BG)
    • Gurdev, Ljuan
      1404 Sofia (BG)
    • Stoyanov, Dimitar
      1172 Sofia (BG)
    • Koch, Vesselinka, Dr.
      5748 Garching (DE)
    • Vankov, Orlin Ivanov
      1164 Sofia (BG)

(74) Representative: Fischer, Michael et al
    Siemens AG,
    Postfach 22 16 34
    80506 München (DE)

(54)  **Apparatus for determining optical and spatial characteristics of an inclusion in a turbid medium using multiple-scattering optical tomography**

(57)  The apparatus allows to determine the optical and spatial characteristics of an inclusion by simply observing the backscattered photon beams. In particular, the evaluation of the light intensity distribution for the backscattered photon beams enables the principal opportunity to use the apparatus for creation of miniature instruments for future applications in medical practice since the instrument can be designed similar to an ultrasonic head.

FIG 1

EP 2 165 647 A1

## Description

The invention relates to an apparatus for determining optical and spatial characteristics of an inclusion in a turbid medium using multiple-scattering optical tomography, especially by using a continuous wave laser.

Imaging through turbid media has become an important field of research, for its great potential in diverse fields of applications like meteorology, ecology, aviation, astrophysics, etc. Currently, emphasis has mostly been laid on medical applications using infrared or visible light instead of harmful X-rays for imaging through tissues. This is referred to as optical tomography. Studies have shown that the absorption and scattering characteristics at a given wavelength by normal tissues differ significantly from the characteristics of other tissues like tumors, kidney stones, etc. This behavior has been adapted for discriminating or locating tumors. Basic limitations are posed by the effect of multiple scattering of light as well as hardware schematics whereby minimizing the multiple scattering effects are difficult to realize.

Most of well known optical tomography techniques, like the double-side optical tomography, are based mainly on a transmission approach for detection of radiation transmitted through the turbid medium, in other words based on a forward scattering method. This method is characterized by double-side schematics, problems with a determination of the optical characteristics of the turbid medium, large measurement times of ballistic and snake photons and an impossibility to distinguish between normal and abnormal tissues.

Other methods for biomedical imaging are using a frequency-domain approach as it is simpler in terms of hardware and feature single sided schematics, however they are characterized by small sounding depths, a very low level of useful signal and a low spatial resolution.

All present solutions are extremely complicated and expensive, using as a rule more than a single high-technology laser source, precise electronics for acquisition and imaging of information, consecutively requiring time-consuming calibration procedures. Moreover, they present ambiguous solutions of corresponding inverse mathematical problems involving the multiple scattering effects.

A turbid medium is a strongly scattering material, for example a tissue. An inclusion is a volume of a different material being a turbid medium of different scattering characteristics enclosed in the strongly scattering material.

Spatial characteristics are to be understood as a totality of information comprising a position of the inclusion relative to a reference point, as well as 3-dimensional coordinates describing a volume of the inclusion.

Optical characteristics describe an interaction of light with the turbid medium and the inclusion, like an absorption coefficient $\mu a$, an integral scattering coefficient $\mu s$, a back scattering coefficient $\beta$, an extinction coefficient $\varepsilon$ being a sum of the absorption coefficient and the integral scattering coefficient, and a scattering angular dependence $\chi(\theta)$ on the angle $\theta$ between an incident and a scattering direction, which is assumed to be sharply forward oriented, meaning that it sharply drops to zero at an angle close to zero.

In this document, the term forward scattering is used for photons which have undergone few scattering events and travel in the direction of the incident radiation under certain angles, less than $\pi/2$ to the incident radiation direction.

The term back scattering, on the contrary, refers to photons which have undergone multiple scattering events and travel in a direction opposite to the incident radiation within a given solid angle.

It is therefore an objective of the present invention to provide an apparatus for determining characteristics of inclusions in a simple way, avoiding the necessity of solving ill-posed mathematical problems and having to use complicated calibration procedures.

These objectives are achieved according to the present invention by an apparatus for determining optical and spatial characteristics of an inclusion in a turbid medium using multiple-scattering optical tomography, comprising:

> a) an array of light sources, each light source emitting a collimated laser incident radiation along an optical axis (z),
> b) a filter array spatially filtering the totality of backscattered secondary photon beams, the filter array being arranged to yield a number of back scattered photon beams having a substantially parallel orientation to the optical axis (z),
> c) an array of detector elements, each detector element detecting an optical output power of said back scattered photon beams,
> d) a control unit for computing the spatial characteristics of the inclusion (2) based on the spatial distribution of the detected optical output power of said back scattered photon beams.

The apparatus therefore allows to determine the optical and spatial characteristics of an inclusion by simply observing the backscattered photon beams. In particular, the evaluation of the light intensity distribution for the backscattered photon beams enables the principal opportunity to use the apparatus for creation of miniature instruments for future applications in medical practice since the instrument can be designed similar to an ultrasonic head.

In order to further significantly decrease any possible efforts with respect to calibration issues or mathematical problems the control unit is preferably further enabled:

> a) to determine a contrast and a sign of said contrast of the inclusion, based on a comparison of the spatial distribution of the optical output power of said back scattered photon beams for the turbid medium (1) without the inclusion (2) and the spatial distribution of the optical output power of said back scattered photon beams for the turbid medium (1) comprising the inclusion (2), and

b) to compute the optical characteristics of the inclusion (2) and of the turbid medium (1) based on the spatial characteristics and the spatial distribution of the optical output power of said backscattered photon beams for the turbid medium (1) comprising the inclusion (2).

A further preferred embodiment of the present invention resides in the construction of the filter array. A preferred filter array may comprise a number of distributed tube-like filter channels, said distributed tube-like filter channels being oriented parallel to each other along the direction of the optical axis (z) in order to reject and/or to absorb all particular backscattered photon beams which lack the substantial parallel orientation to the optical axis (z). Furthermore, said filter channels may be formed as cylindrical apertures within a filter block, said cylindrical apertures being oriented parallel to the optical axis (z).

In order to provide a rather simple design for the collimated laser incident radiation, each light source preferably emits its collimated laser incident radiation along the optical axis, each of the radiations originating from a different continuous wave laser.

In a further preferred embodiment of the present invention the filter array may additionally host the array of light sources, thereby comprising an even periodical distribution of the detector elements and light sources as seen in the cross-sectional area oriented perpendicular to the optical axis. Even distribution in the context does not mean an equal periodicity of both the detector elements and the light sources but that both the detector elements and light sources have a regular periodicity but notably not the same periodicity. Therefore, the number of detector elements may be at least twice as big as the number of light sources. I.e., the detector elements and the light elements are disposed in regular chessboard-like pattern whereby each light source comprises at least four exclusive detector elements juxtaposed, preferably eight exclusive detector elements.

In order to improve the spatial resolution of the apparatus, the light sources are preferably controlled by the control unit; said control unit allowing the light sources to emit one after the other and only one at a time. Accordingly, the control unit is preferably collecting the detected optical output power in response to each of the emitted multiple radiations.

Further preferred embodiment may be derived from the remaining dependent claims.

Preferred examples of the present invention are hereinafter described in more detail with reference to the following illustrative and not-limiting drawings, which depict in:

Figure 1     a general schematic block diagram of a one-dimensional apparatus for optical tomography;

Figure 2     a general schematic block diagram of an apparatus for optical tomography with a linear filter array incorporating also a linear array of light sources;

Figure 3     schematically a cross-sectional view on a filter array incorporating also an array of light sources;

Figure 4     the basic principle to the measurement of the light intensity of a backscattered photon beam;

Figure 5     a possible 2D implementation of the block of lasers in the matrix in Fig.3 and in Fig.4;

Figure 6     a possible realization of an optical multi-detection unit of an optical multi-detection and measurement system;

Figure 7     schematically a structural implementation of an apparatus for optical tomography; and

Figure 8     schematically a realization for an apparatus for optical tomography.

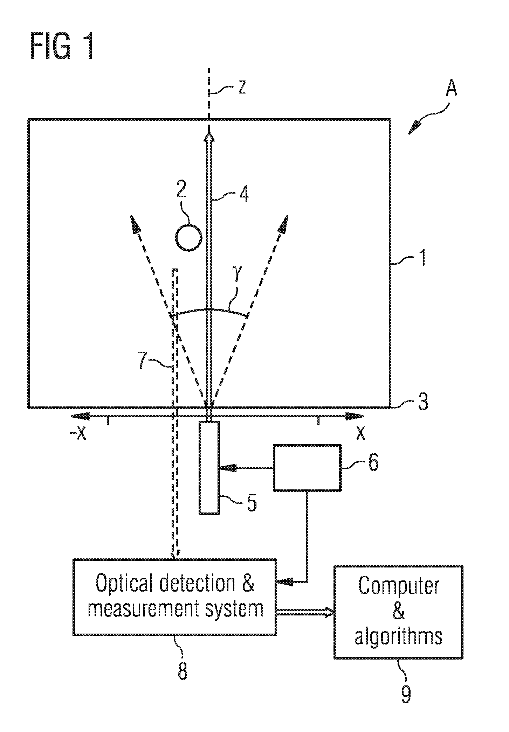

The general block-schematic of an one-dimensional (1D) apparatus A for optical tomography, preferably based on the method for optical tomography as filed with the European Patent application 08014407.4 on August 13, 2008 which is incorporated herein by reference and which priority is currently claimed is given in Fig.1. According to Fig.1, a turbid medium 1, containing (or not containing) an inclusion 2 is illuminated through a contact surface 3 in the coordinate point $x=0$ by a collimated optical beam 4 emitted along an optical axis $z$ from a laser source 5, driven by the laser driver 6. A specific backscattered photon beam 7, which is parallel to the optical axis $z$, is optically linked after passing the contact surface 3 through an optical detection and measurement block 8 with a computer and algorithms block 9.

The operation of the apparatus A in Fig.1, based on the method for optical tomography mentioned above is as follows. The input collimated optical radiation 4 begins to scatter immediately after entering the turbid medium 1 through the contact surface 3. As a result of the multiple scattering, the turbid medium 1 containing the inclusion 2 will be illuminated within a spread angle $\gamma$. A part of this radiation 4, backscattered in parallel to the optical axis $z$ contains, after passing the contact surface 3, the specific photon beam 7, which is then optically detected and measured by the optical detection and measurement block 8. The amplitude of the output signal

(at $x$ coordinates $j_x = 0$ of the laser beam, $k_x$ of the specific photon beam 7 and $p_x$ of the inclusion 2, at a depth $p_z$ of the inclusion) can be denoted by $S_{j_x=0}(k_x, p_x, p_z)$, in the presence of an inclusion, and $S^0_{j_x=0}(k_x)$, in the absence of any inclusions

In the last expressions the output signal is represented as depending on the discrete coordinates $k_x$, along the $x$ - axis of the positions of the specific photon beams 7, given by $x_k = k_x \Delta_x$, where $k_x = 1...K_x$, and $K_x \Delta_k$ is the maximum tolerable distance between the laser beam 4 and the specific backscattered photon beams 7. The step $\Delta_k$ is defined as the minimum distance between the adjacent non-overlapped specific photon beams along the $x$ -axis.

By disposing the optical detector in the optical detection and measurement block 8 in $K_x$ successive positions $k_x$ along the $x$ - axis, one can create the set of output signals $\{S_{j_x=0}(k_x, p_x, p_z)\}_{k_x}$. Also, one can vary the dispositions of the laser beam in $J_x$ successive points $j_x, \Delta_j$ ($j_x = 0...J_x$) along the $x$ - axis of step $\Delta_j = m\Delta_k$, where $m > 1$ is an integer. So, one can create a set of output signals for each position of the laser beam. This set can be denoted by $\{S_{j_x}(k_x, p_x, p_z)\}_{k_x, j_x}$ in the presence of an inclusion, and by $\{S_{j_x}(k_x)\}_{k_x, j_x}$ in the absence of any inclusions.

Then, according to the method for optical tomography, one can calculate (in block 9) the coordinates of the inclusion 2 and the optical characteristics of the turbid medium 1 and the inclusion 2.

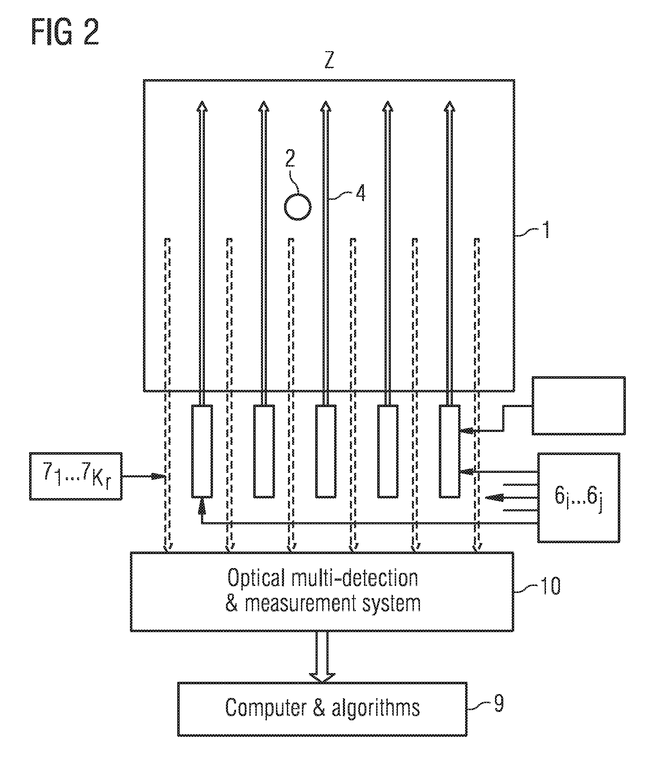

Further considering now in Fig.2 a set of $K_x$ parallel specific backscattered beams $7_1.....7_{K_x}$, disposed at the corresponding positions as in Fig.1, each one linked to a corresponding detector of the set of $K_x$ optical detectors in an optical multi-detection and measurement system 10, and a set of $J_x$ emitting lasers $5_1..,5_{J_x}$, disposed also along the $x$ - axis with a step $\Delta_j = m\Delta_k$, $m > 1$, each one driven by a corresponding driver of the set $6_1...6_{J_x}$ in the block 6.

The operation of the apparatus in Fig.2, based on the method for optical tomography and the apparatus A in Fig.1 is as follows. In each moment one of the emitting lasers (say, $j_x$) of the set $5_1...5_{J_x}$ is switched to an "ON" state, while all other lasers are kept in an "OFF" state. The optical detectors in the optical multi-detection and measurement system 10 are always in the "ON" condition. Each of these optical detectors will measure the optical powers from the corresponding specific backscattered photon beams $7_1.....7_{K_x}$. In this way the entire set of output signals $\{S_{j_x}(k_x, p_x, p_z)\}_{k_x}$ can be measured, corresponding to some defined position of the emitting laser in the Fig.1, without successive positioning of any single optical detector in the optical detection and measurements system 8.

Then, the emitting laser is switched "OFF" and the next laser $j_x+1$ will switch "ON", while all other lasers remain in "OFF" state. The optical detectors in the optical multi-detection and measurement system 10 remain in the "ON" state. The recorded set of output signals for this case will be $\{S_{j_x+1}(k_x, p_x, p_z)\}_{k_x}$. As evident, the entire set of output signals $\{S_{j_x}(k_x, p_x, p_z)\}_{k_x, j_x}$ in the presence of an inclusion, and $\{S_{j_x}(k_x)\}_{k_x, j_x}$ in the absence of any inclusions, that is necessary to calculate (block 9) the coordinates of the inclusion 2 and the optical characteristics of the turbid medium 1 and the inclusion 2, will be created after successive switching in "ON" state the entire set of emitting lasers $5_1...5_{J_x}$. At this implementation all the elements of the apparatus remain unmoved. Moreover, the full measurement time can be thus minimized.

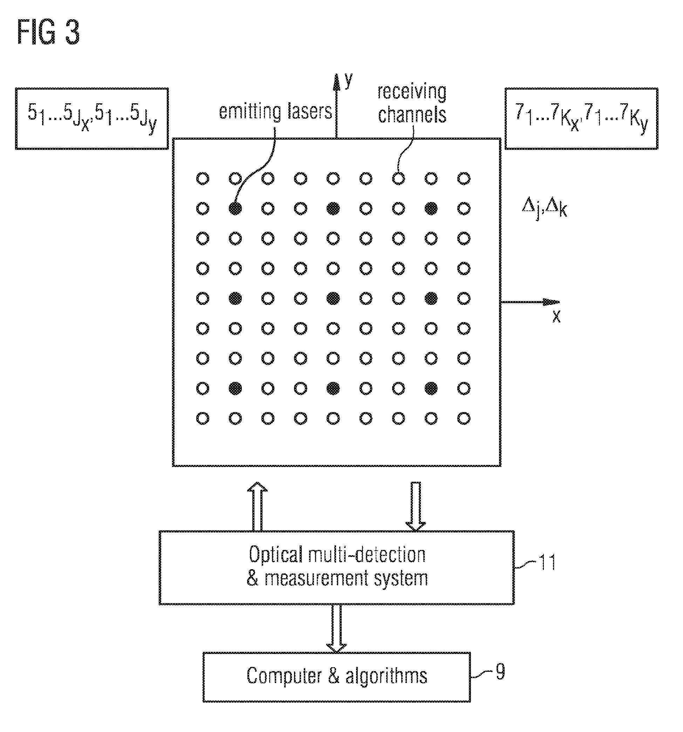

In the following a 2D-implementation of the apparatus for optical tomography according the Fig.1 and Fig.2 is considered. It is given as a cross-sectional view in Fig.3, where the disposition of the two dimensional set (a matrix) of lasers $5_1...5_{J_x}, 5_1,...5_{J_y}$ and a matrix of specific backscattered photon beams $7_1...7_{K_x}, 7_1...7_{K_y}$ on the contact surface is represented. According to Fig.3, the space step $\Delta_j$ of the laser dispositions along both the coordinates is chosen to be larger ($\Delta_j = 3\Delta_k$) than the step $\Delta_k$ of specific backscattered photon beams $7_1...7_{K_x}$, $7_1...7_{K_y}$. Moreover, the two-dimensional optical multi-detector system 11 contains a two-dimensional set of optical detectors, each one linked to the corresponding specific backscattered photon beams of the set $7_1...7_{K_x}, 7_1...7_{K_y}$.

The operation of the two dimensional apparatus in Fig.3 is similar to that of the one dimensional apparatus in Fig.2. In each moment one of the lasers in the laser matrix $5_1...5_{J_x}, 5_1...5_{J_y}$ is in "ON" state, while all other lasers are switched to "OFF" state. Then, the next laser in the matrix will be switched to "ON" state, while all other lasers are switched to "OFF" state. The two dimensional matrix $7_1...7_{K_x}, 7_1...7_{K_y}$ of optical detectors remains always in "ON" state. As evident, the entire set of output signals will be given now by

$$\{S_{j_x, j_y}(k_x, k_y, p_x, p_y, p_z)\}_{k_x, k_y, j_x, j_y} \quad \text{in the presence}$$

of an inclusion, and by $\{S^0_{j_x, j_y}(k_x, k_y)\}_{k_x, k_y, j_x, j_y}$ in the absence of any inclusions.

This is the necessary set of signals to calculate (block 9) the 3D coordinates (two lateral ones and the depth) of the inclusion and the optical characteristics of the medium 1 and the inclusion 2. At this implementation all elements of the apparatus remain unmoved. Moreover, the full measurement time can be thus minimized.

The numbers $N_{lasers}$ of lasers in $5_1...5_{J_x},5_1...5_{J_y}$ and $N_{det}$ of optical detectors in $7_1...7_{K_x},7_1...7_{K_y}$ of the matrix in Fig.3 can be determined by the following expressions (assuming symmetric implementation along both the lateral coordinates):

$$N_{lasers} = (2L+1)^2 ,$$

$$N_{det} = 4(M-L)(M+L+1) ,$$

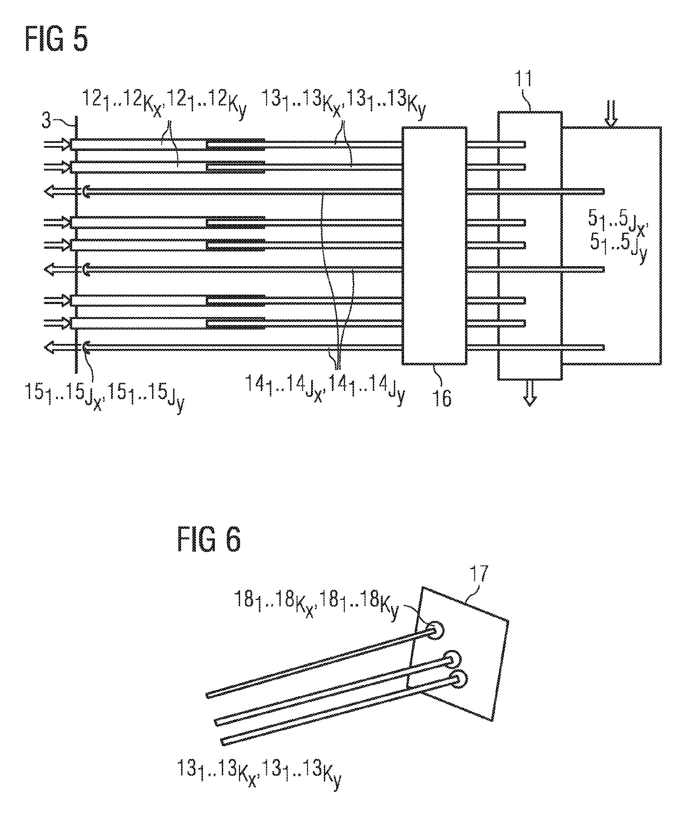

where $L$ is the number of lasers along one of the axes from the coordinate origin to the end of the matrix, $M$ is the total number of lasers plus detectors along one of the axis from the coordinate origin to the end of the matrix. According to Fig.5 ($L = 1$, $M = 6$) one will obtain $N_{lasers}$ = 9 and $N_{det}$ = 169.

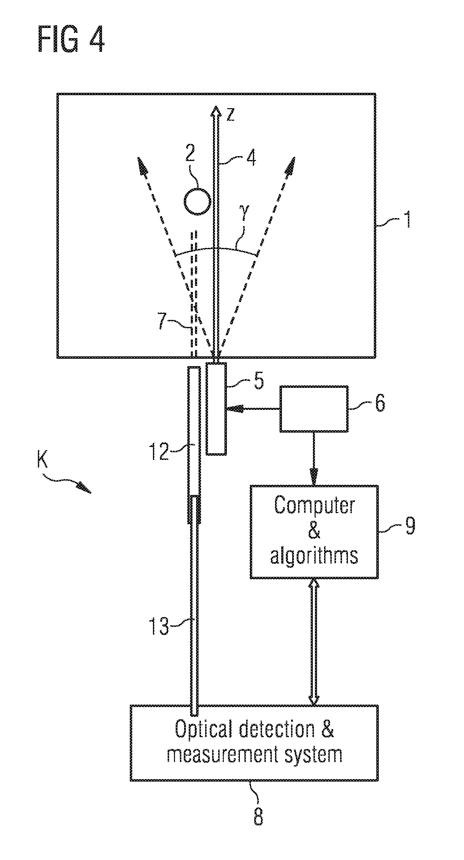

Fig.4 represents one possible implementation of the key block K, providing forming of the specific backscattered photon beam 7, which is parallel to the optical axis z. According to Fig.4, the formation of the backscattered photon beam 7 is realized by spatial filtering of scattered radiation using a tiny cylindrical tube 12 of internal diameter $d_{tube}$ and length $l_{tube}$ and an optical fiber 13. The tiny tube 12 is disposed with one of its edges near the laser beam 4 with an axis, parallel to the laser beam. The optical fiber 13 is inserted with one of its edges into the opposite (rear) edge of the tube 12, while the other fiber edge is fed to the optical detector of the optical detection & measuring system 8.

The length $l_{tube}$ of the tube 12 is assumed to be too long with respect to the internal diameter $d_{tube}$, i.e. $d_{tube} \ll l_{tube}$. The basic function of tubes is to provide the very narrow field of view for the photon detection. The tube angle of view $\theta_{tube}$ could be approximately determined by the expression $\theta_{tube} \sim d_{tube}/l_{tube}$ (say, for $d_{tube} \sim 0.1$ cm and $l_{tube} \sim 5$ cm we obtain that the tube field of view will be of the order of $\theta_{tube} \sim 20$ mrad). For an inclusion depths $p_z \sim 6$ cm, the lateral receiving spot diameter will be of the order of 1.5 mm and thus, the overlapping of adjacent specific backscattered photon beams 7 can be easily avoided. Under these conditions one could neglect the mutual penetrations of the scattered light from adjacent parallel cylindrical volumes. The angular filtering can be improved by deposition of some absorbing coating on the internal wall of the tube 12. Moreover, for increasing the photon collection inside the tube 12, one can modify the receiving fibre edge surface, say, by forming a lens surface. The optical energy, passing through the tube 12 and the fiber 13 will create the signals, necessary to determine the position of the inclusion 2 and the optical characteristics of the turbid medium 1 and the inclusion 2.

The set of specific backscattered photon beams for the 2D implementation of the apparatus can be formed (according to Figures 2 and 3) by a set of parallel tiny tubes $12_1...12_{K_x},12_1...12_{K_y}$ with corresponding optical fibers $13_1...13_{K_x},13_1...13_{K_y}$. The outputs of optical fibers $13_1...13_{K_x},13_1...13_{K_y}$ are fed to corresponding optical detectors, disposed within the multi-channel optical detection and measurements system 11. The distance between the adjacent tiny tubes 12 will be equal to the step of the matrix in Fig.3. In this case the matrix in Fig. 3 is formed by the open edges of tubes $12_1...12_{K_x},12_1...12_{K_y}$ and lasers $5_1...5_{J_x},5_1...5_{J_y}$. At these configurations the full set of output signals

$$\left\{ S_{j_x,j_y}(k_x,k_y,p_x,p_y,p_z) \right\}_{k_x,k_y,J_x,J_y} \text{ or}$$

$$\left\{ S^0_{j_x,j_y}(k_x,k_y) \right\}_{k_x,k_y,J_x,J_y} \text{ will be measured.}$$

Fig.5 presents a possible 2D implementation of the block of lasers in the matrix in Fig.3 and in Fig.4. According to Fig.5, the emitted radiation from the two-dimensional block of lasers $5_1...5_{J_x},5_1...5_{J_y}$ (as in Fig.3) is directed into the optical fibers $14_1...14_{J_x},14_1...14_{J_y}$. Lenses $15_1...15_{J_x},15_1...15_{J_y}$ are disposed on the opposite fiber edges (in some other configuration the lenses $15_1...15_{J_x},15_1...15_{J_y}$ are implemented on the fiber edges). The use of lenses 15 provides an emission of collimated laser beams into the turbid medium 1.

The optical bunch 16 contains the fibers $13_1...13_{K_x},13_1...13_{K_y}$ of the receiving part of the apparatus and the fibers $14_1...14_{J_x},14_1...14_{J_y}$ of the emitting part. The optical detectors within the multi-channel optical detection and measurement system 11 are correspondingly linked with the fibers $13_1...13_{K_x},13_1...13_{K_y}$. Moreover, the optical fibers $14_1...14_{J_x},14_1...14_{J_y}$ in the emitting part are correspondingly linked with the lasers $5_1...5_{J_x}5_1...5_{J_y}$.

The configuration of the apparatus in Fig.5 provides electrical separation of the apparatus as a whole from the contact surface 3 that is connected mechanically with the patient, in particular which is the skin of the patient.

One possible realization of the optical multi-detection unit of the optical multi-detection and measurement system 11 is shown in Fig.6. Here the properly in space arranged set of edges of fibers $13_1...13_{K_x},13_1...13_{K_y}$ illuminates a surface 17 of a photosensitive matrix (say, CCD-matrix, intensifier photosensitive surface, etc.). The special arrangement of fibers 13 is chosen by the condition that the corresponding light spots $18_1...18_{K_x},18_1...18_{K_y}$, created by each of the fibers 13 be non-overlapped. Under these conditions the set of optical detectors in the optical multi-detection measurement system 11 is replaced by a single photosensitive matrix. The energies in the set of light spots on the single photosensitive matrix are proportional to the corresponding output

signals $\left\{ \bar{S}_{j_x,j_y} \left( \bar{k}_x, \bar{k}_y, p_x, p_y, p_z \right) \right\}_{k_x,k_y,j_x,j_y}$ or

$\left\{ \bar{S}^0_{j_x,j_y} \left( \bar{k}_x, \bar{k}_y \right) \right\}_{k_x,k_y,j_x,j_y}$, that are necessary to determine the coordinates of the inclusion 2 and the optical characteristics of the turbid medium 1 and the inclusion 2. Measuring, by the use of CCD-optical receiver the set of above signals, one can essentially reduce the implementation of the apparatus.

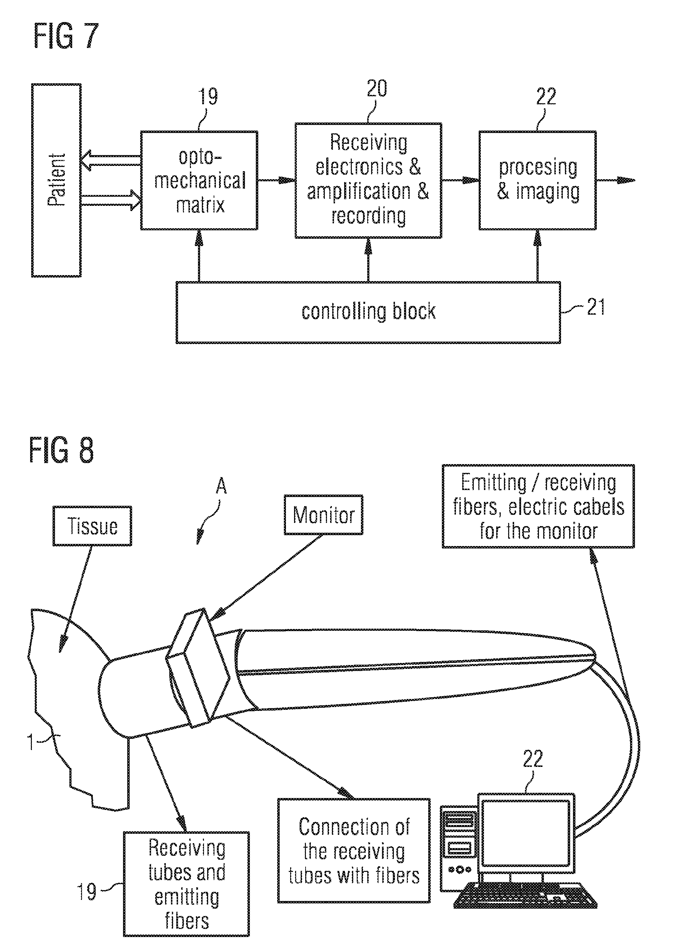

One of the implementations of the apparatus for optical tomography can be realized using the block-schematic in Fig. 7. Here the optomechanical matrix (according to realizations in Fig.5 and Fig.6 ) is in contact with patients. It is well known that the patient breast tissue can be considered as a turbid medium. The optomechanical matrix 19 illuminates the patient breast tissue (containing, say, some cancer-type inclusions). The backscattered radiation formed in a set of parallel photon beams (see Fig.4 and Fig.5) is transformed (see Fig.6) in a set of arranged light spots of defined energies. Then, the set of spots is transformed further by a receiving electronics/amplification/recording block 20 into a set of signals. The recorded set of signals

$\left\{ S_{j_x,j_y} \left( k_x, k_y, p_x, p_y, p_z \right) \right\}_{k_x,k_y,j_x,j_y}$ or

$\left\{ S^0_{j_x,j_y} \left( k_x, \bar{k}_y \right) \right\}_{k_x,k_y,j_x,j_y}$ is then processed by a processor block 22. The output results are imaged at least on a monitor together with the information about the inclusion coordinates and their optical characteristics. This information is further processed in order to determine the specificity of the inclusions.

Then, one possible implementation of the apparatus A as a whole is given in Fig.8 for illustration. The basic block of this apparatus demonstrates the principal opportunity to use both the method and the apparatus for creation of miniature instruments for future applications in medical practice.

## Claims

1. Apparatus (A) for determining optical and spatial characteristics of an inclusion (2) in a turbid medium (1) using multiple-scattering optical tomography, comprising:

   a) a matrix of light sources (5), each light source (5) emitting a collimated laser incident radiation (4) along an optical axis (z),
   b) a filter matrix (K, 12, 13) spatially filtering a totality of backscattered secondary photon beams (7), the filter matrix (K, 12, 13) being arranged to yield a number of back scattered photon beams having a substantially parallel orientation to the optical axis (z),
   c) an array (17) of detector elements, each detector element detecting an optical output power of said back scattered photon beams (7),
   d) a control unit (9, 21, 22) for computing the spatial characteristics of the inclusion (2) based on the spatial distribution of the detected optical output power of said back scattered photon beams (7).

2. Apparatus (A) according to claim 1, whereby the control unit (9, 21, 22) is further enabled:

   a) to determine a contrast and a sign of said contrast of the inclusion (2), based on a comparison of the spatial distribution of the optical output power of said back scattered photon beams (7) for the turbid medium (1) without the inclusion (2) and the spatial distribution of the optical output power of said back scattered photon beams (7) for the turbid medium (1) comprising the inclusion (2), and
   b) to compute the optical characteristics of the inclusion (2) and of the turbid medium (1) based on the spatial characteristics and the spatial distribution of the optical output power of said backscattered photon beams (7) for the turbid medium (1) comprising the inclusion (2).

3. Apparatus (A) according to claim 1 or 2, whereby the filter matrix (K, 12, 13) comprises a number of distributed tube-like filter channels (12), said distributed tube-like filter channels (12) being oriented parallel to each other along the direction of the optical axis (z) in order to reject and/or to absorb all particular backscattered photon beams which lack the substantial parallel orientation to the optical axis (z).

4. Apparatus (A) according to claim 3, whereby said filter channels are formed as cylindrical apertures (12) within a filter block (K), said cylindrical apertures (12) being oriented parallel to the optical axis (z).

5. Apparatus (A) according to any of the preceding claims, wherein each light source (5) emits its collimated laser incident radiation (4) along the optical axis (z), each of the radiations originating from a different continuous wave laser (6).

6. Apparatus (A) according to claim 5, wherein the filter matrix hosts the array of light sources (5), thereby comprising an even distribution of the detector elements and light sources (5) as seen in the cross-sectional area oriented perpendicular to the optical axis (z).

7. Apparatus (A) according to claim 6, wherein the number of detector elements is at least twice as big as the number of light sources (5).

8. Apparatus (A) according to claim 6 or 7, wherein the detector elements and the light sources (5) are disposed in regular chessboard-like pattern whereby each light source (5) comprises at least four exclusive detector elements juxtaposed.

9. Apparatus (A) according to claim 6, 7 or 8, wherein the output light spots of the tube-like filter channels (12) are spatially arranged in a non-overlapping configuration and optically linked to a corresponding array of detector elements (17) formed as non-overlapping areas of adjacent pixels of a single photosensitive matrix.

10. Apparatus (A) according to any of the preceding claims, wherein the light sources (5) are controlled by the control unit (9, 21, 22); said control unit (9, 21, 22) allowing the light sources (5) to emit one after the other and only one at a time.

11. Apparatus (a) according to claim 10, wherein the control unit (9, 21, 22) is collecting the detected optical output power in response to each of the emitted collimated laser incident radiations (4).

## FIG 1

FIG 2

Z

2

4

1

$7_1 ... 7K_r$

$6_i ... 6_j$

Optical multi-detection
& measurement system

10

Computer & algorithms

9

# FIG 3

$5_1...5_{J_X}, 5_1...5_{J_y}$

emitting lasers

receiving channels

$7_1...7_{K_X}, 7_1...7_{K_y}$

$\Delta_j, \Delta_k$

y

x

Optical multi-detection & measurement system — 11

Computer & algorithms — 9

FIG 4

## FIG 5

$12_1..12_{K_x},12_1..12_{K_y}$   $13_1..13_{K_x},13_1..13_{K_y}$

11

3

$5_1..5_{J_x},$
$5_1..5_{J_y}$

$15_1..15_{J_x},15_1..15_{J_y}$

$14_1..14_{J_x},14_1..14_{J_y}$

16

## FIG 6

17

$18_1..18_{K_x},18_1..18_{K_y}$

$13_1..13_{K_x},13_1..13_{K_y}$

## FIG 7

## FIG 8

Europäisches
Patentamt

European
Patent Office

Office européen
des brevets

**EUROPEAN SEARCH REPORT**

Application Number

EP 08 01 9605

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 948 974 A (NELSON ROBERT S [US] ET AL) 14 August 1990 (1990-08-14) <br> * column 2, lines 17-20,23-26,54-60 * <br> * column 3, lines 2-4,14-16,40-45,50-55,57-62 * <br> * column 4, line 30 - column 5, line 9 * <br> * column 5, lines 52-56 * <br> * column 6, lines 16-22,33-44,52-56 * <br> * column 7, line 49 - column 8, line 9 * <br> * column 9, lines 43-49 * <br> * figures 2(b),3,7,8 * | 1-9 | INV. <br> A61B5/00 <br><br> ADD. <br> G01N21/47 <br> G01N21/49 |
| Y | | 10,11 | |
| Y | US 5 747 789 A (GODIK EDUARD E [US]) 5 May 1998 (1998-05-05) <br> * column 7, line 49 - column 8, line 9 * <br> * column 9, lines 43-49 * <br> * figure 3 * <br> ----- | 10,11 | |
| | | | TECHNICAL FIELDS SEARCHED (IPC) <br><br> A61B <br> G01N |

The present search report has been drawn up for all claims

| Place of search | Date of completion of the search | Examiner |
|---|---|---|
| The Hague | 10 March 2009 | Faymann, Juan |

EPO FORM 1503 03.82 (P04C01)

**EP 2 165 647 A1**

## ANNEX TO THE EUROPEAN SEARCH REPORT
## ON EUROPEAN PATENT APPLICATION NO.

EP 08 01 9605

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2009

| Patent document cited in search report | | Publication date | Patent family member(s) | Publication date |
|---|---|---|---|---|
| US 4948974 | A | 14-08-1990 | NONE | |
| US 5747789 | A | 05-05-1998 | NONE | |

EPO FORM P0459

For more details about this annex : see Official Journal of the European Patent Office, No. 12/82

15

**EP 2 165 647 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 08014407 A

16